(19) Europäisches Patentamt
European Patent Office
Office européen des brevets

(11) EP 0 783 882 B1

(12) **EUROPÄISCHE PATENTSCHRIFT**

(45) Veröffentlichungstag und Bekanntmachung des Hinweises auf die Patenterteilung:
**22.06.2005 Patentblatt 2005/25**

(51) Int Cl.[7]: **A61K 7/42**, A61K 7/00

(21) Anmeldenummer: **96119491.7**

(22) Anmeldetag: **05.12.1996**

(54) **Kosmetische und dermatologische Lichtschutzformulierungen in Form von Emulsionen**

Cosmetic or dermatological photoprotective emulsions

Emulsions cosmétiques et dermatologiques pour la protection solaire

(84) Benannte Vertragsstaaten:
**AT BE CH DE ES FR GB IT LI NL**

(30) Priorität: **21.12.1995 DE 19548016**

(43) Veröffentlichungstag der Anmeldung:
**16.07.1997 Patentblatt 1997/29**

(73) Patentinhaber: **Beiersdorf Aktiengesellschaft**
**20245 Hamburg (DE)**

(72) Erfinder:
- **Gers-Barlag, Heinrich, Dr.**
  **25495 Kummerfeld (DE)**
- **Kröpke, Rainer**
  **22527 Hamburg (DE)**

(56) Entgegenhaltungen:
**EP-A- 0 281 394**
**EP-A- 0 457 687**
**EP-A- 0 685 221**
**EP-A- 0 685 223**
**EP-A- 0 685 224**
**DE-A- 4 337 041**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 783 882 B1

## Beschreibung

Die vorliegende Erfindung betrifft kosmetische und dermatologische Lichtschutzzubereitungen, insbesondere hautpflegende kosmetische und dermatologische Lichtschutzzubereitungen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutze gegen UVB-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubstanzen zur Verfügung zu haben, da auch dessen Strahlen Schäden hervorrufen können. So ist erwiesen, daß UVA-Strahlung zu einer Schädigung der elastischen und kollagenen Fasern des Bindegewebes führt, was die Haut vorzeitig altem läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, z.B. Hydroxylradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurziebige Epoxide und viele Andere. Singulettsauerstoff beispielsweise zeichnet sich gegenüber dem normalerweise vorliegenden Triplettsauerstoff (radikalischem Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Ein vorteilhafter UVB-Filter ist der 4,4',4''-(1,3,5-Triazin-2,4,6-triyltriimino)-tris-benzoesäure-tris(2-ethylhexylester), synonym: 2,4,6-Tris-[anilino-(p-carbo-2'-ethyl-1'-hexyloxy)]-1,3,5-triazin.

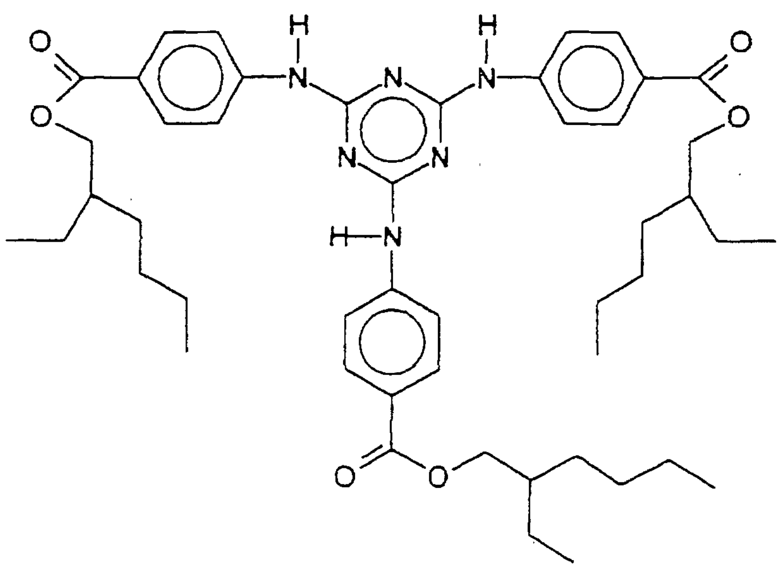

Diese UVB-Filtersubstanz wird von der BASF Aktiengesellschaft unter der Warenbezeichnung UVINUL® T 150 vertrieben und zeichnet sich durch gute UV-Absorptionseigenschaften aus.

Der Hauptnachteil dieses UVB-Filters ist die schlechte Löslichkeit in Lipiden. Bekannte Lösungsmittel für diesen UVB-Filter können maximal ca. 15 Gew.-% dieses Filters lösen, entsprechend etwa 1 - 1,5 Gew.-% gelöster, und damit aktiver, UV-Filtersubstanz.

Auch andere schwerlösliche UV-Filtersubstanzen sind bekannt, beispielsweise 2-Phenylbenzimidazol-5-sulfonsäure bzw. ihre Salze, insbesondere das Natrium-, das Kalium und das TEA-Salz, beispielsweise erhältlich unter der Bezeichnung Eusolex® 232 der Merck AG, welches sich durch folgende Strukturformel auszeichnet:

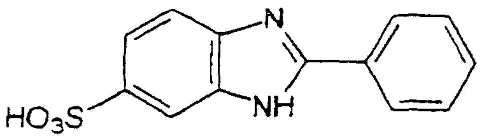

Selbst wenn grundsätzlich ein gewisser UV-Schutz bei gegebener begrenzten Löslichkeit, (und damit nach herkömmlichen Maßstäben: schlechten Einarbeitbarkeit in eine kosmetische oder dermatologische Zubereitung) erreicht werden kann, kann ein anderes Problem auftreten, die Rekristallisation. Diese tritt gerade bei schlecht löslichen Substanzen vergleichsweise schnell ein, sei es durch Temperaturschwankungen oder andere Einflüsse hervorgerufen. Unkontrollierte Rekristallisation eines wesentlichen Zubereitungsbestandteiles wie eines UV-Filters hat aber äußerst nachteilige Einwirkungen auf die Eigenschaften der gegebenen Zubereitung und, nicht zuletzt, auf den angestrebten Lichtschutz.

Es ist ferner bekannt, daß sich multiple Emulsionen - unter anderem - durch eine besonders feine Emulsionstextur auszeichnen können. Diese Eigenschaft eignet sie hervorragend als Basis sowohl für kosmetische wie für medizinische topische Zubereitungen.

In einfachen Emulsionen liegen in der einen Phase feindisperse, von einer Emulgatorhülle umschlossene Tröpfchen der zweiten Phase (Wassertröpfchen in WO- oder Lipidvesikel in O/W-Emulsionen) vor. In einer multiplen Emulsion (zweiten Grades) hingegen sind in solchen Tröpfchen feiner disperse Tröpfchen der ersten Phase emulgiert. Auch in diesen Tröpfchen wiederum können noch feiner disperse Tröpfchen vorliegen (multiple Emulsion dritten Grades) und so fort.

So wie man also bei den einfachen Emulsionen von W/O- oder O/W-Emulsionen spricht (Wasser-in-Oel oder Oelin-Wasser), gibt es bei multiplen Emulsionen W/O/W-, O/W/O-, O/W/O/W-, W/O/W/O-Emulsionen und so fort.

Multiple Emulsionen, bei welchen die jeweiligen inneren und äußeren Wasserphasen oder inneren und äußeren Ölphasen unterschiedlich geartet sind (also z.B. W/O/W'- und O/W/O' -Emulsionen), sind der Präparation durch Zweitopfverfahren zugängig. Solche Emulsionen, in welchen die inneren und äußeren Wasser- bzw. Ölphasen nicht unterschiedlich geartet sind, sind sowohl durch Ein- als auch durch Zweitopfverfahren erhältlich.

Die multiplen Emulsionen zweiten Grades werden gelegentlich als "bimultiple Systeme", solche dritten Grades als "trimultiple Systeme" usw., bezeichnet (W.Seifriz, Studies in Emulsions, J.Phys.Chem., 29 (1925) 738 - 749).

Verfahren zur Herstellung multiplet Emulsionen sind dem Fachmann an sich geläufig. So gibt es Zweitopfverfahren, in welchen eine einfache Emulsion (z.B. eine W/O-Emulsion) vorgelegt und durch Zugabe einer weiteren Phase (z.B. eine Wasserphase) mit einem entsprechenden Emulgator (z.B. ein O/W-Emulgator) in eine multiple Emulsion (z.B. eine W/O/W- Emulsion) überführt wird.

Eine zweites bekanntes Verfahren besteht darin, Emulgatorgemische mit einer Ölphase und einer Wasserphase in einem Eintopfverfahren in eine multiple W/O/W-Emulsion zu überführen. Die Emulgatoren werden in der Ölphase gelöst und mit der Wasserphase vereinigt. Voraussetzung für ein solches Verfahren ist, daß die HLB-Werte (HLB = Hydrophil-Lipophil-Balance) der eingesetzten einzelnen Emulgatoren sich deutlich voneinander unterscheiden.

Die Definition für den HLB-Wert ist für Polyolfettsäureester gegeben durch die Formel I

$$HLB = 20 * (1 - S/A)$$

Für eine Gruppe von Emulgatoren, deren hydrophilen Anteil nur aus Ethylenoxideinheiten besteht, gilt die Formel II

$$HLB = E/5$$

wobei

S = Verseifungszahl des Esters,
A = Säurezahl der zurückgewonnen Säure
E = Massenanteil Ethylenoxid (in %) am Gesamtmolekül

bedeuten.

Emulgatoren mit HLB-Werten von 6-8 sind im allgemeinen W/O-Emulgatoren, solche mit HLB-Werten von 8-18 sind im allgemeinen O/W-Emulgatoren.

Literatur: "Kosmetik - Entwicklung, Herstellung und Anwendung kosmetischer Mittel"; W.Umbach (Hrsg.), Georg Thieme Verlag 1988.

Hydrophile Emulgatoren (mit hohen HLB-Werten) sind in der Regel O/W-Emulgatoren. Demgemäß sind hydrophobe oder lipophile Emulgatoren (mit niedrigen HLB-Werten) in der Regel W/O-Emulgatoren.

Die US-Patentschrift 4,931,210 beschreibt ein Verfahren zur Herstellung von W/O/W-Emulsion, wobei als Emulgatoren Polyglycerinpolyricinoleate verwendet werden.

Die Tröpfchendurchmesser der gewöhnlichen "einfachen", also nichtmultiplen Emulsionen liegen im Bereich von ca 1 µm bis ca. 50 µm. Solche "Makroemulsionen" sind, ohne weitere färbende Zusätze, milchigweißgefärbt und opak. Feinere "Makroemulsionen", deren Tröpfchendurchmesser im Bereich von ca. $10^{-1}$ µm bis ca. 1 µm liegen, sind, wiederum ohne färbende Zusätze, bläulichweißgefärbt und opak. Solche "Makroemulsinen" haben für gewöhnlich hohe Viskosität.

Mizellaren und molekularen Lösungen mit Partikeldurchmessern kleiner als ca. $10^{-2}$ µm, die allerdings nicht mehr als echte Emulsionen aufzufassen sind, ist vorbehalten, klar und transparent zu erscheinen.

Der Tröpfchendurchmesser von Mikroemulsionen dagegen liegt im Bereich von etwa $10^{-2}$ µm bis etwa $10^{-1}$ µm. Mikroemulsionen sind transluzent und meist niedrigviskos. Die Viskosität vielen Mikroemulsionen vom O/W-Typ ist vergleichbar mit der des Wassers.

Vorteil von Mikroemulsionen ist, daß in der dispersen Phase Wirkstoffe wesentlich feiner dispers vorliegen können als in der dispersen Phase von "Makroemulsionen". Ein weiterem Vorteil ist, daß sie aufgrund ihrer niedrigen Viskosität versprühbar sind. Werden Mikroemulsionen als Kosmetika verwendet, zeichnen sich entsprechende Produkte durch hohe kosmetische Eleganz aus.

Es ist bekannt, daß hydrophile Emulgatoren bei steigender Temperatur ihr Löslichkeitsverhalten von wasserlöslich zu fettlöslich ändern. Der Temperaturbereich, in dem die Emulgatoren ihre Löslichkeit geändert haben, wird Phaseninversionstemperaturbereich (PIT) genannt.

S.Matsumoto (Journal of Colloid and Interface Science, Vol. 94, No.2, 1983) beschreibt, daß die Entwicklung einer W/O/W-Emulsion einer Phaseninversion konzentrierten W/O-Emulsionen, welche durch Span 80, einen ausgeprägten W/O-Emulgator, stabilisiert sind, vorausgeht. Matsumoto geht dabei von einem extrem unpolaren Öl, nämlich flüssigem Paraffin aus. Darüberhinaus sei eine gewisse Menge hydrophilen Emulgatoren zur Entwicklung einer W/O/W-Emulsion aus einer W/O-Emulsion nötig.

T.J.Lin, H.Kurihara und H.Ohta (Journal of the Society of Cosmetic Chemists 26, S. 121 - 139, März 1975 zeigen bei unpolaren Ölen, daß im Bereich der PIT extrem instabile multiple Emulsionen vorliegen können.

Es hat sich überraschenderweise gezeigt, und darin liegt die Lösung der Aufgaben begründet, daß Verfahren zur Einarbeitung von 4,4',4"-(1,3,5-Triazin-2,4,6-triyltriimino)-tris-benzoesäure-tris(2-ethylhexylester) in O/W-Emulsionen, insbesondere O/W-Mikroemulsionen, dadurch gekennzeichnet, daß

- die Bestandteile einer Wasserphase,
- gegebenenfalls übliche, in Wasser lösliche oder dispergierbare Substanzen,
- die Bestandteile einer Ölphase
- mindestens ein Emulgator (Emulgator A) dessen Lipophilie von der Temperatur abhängig ist, dergestalt, daß die Lipophilie mit steigender Temperatur zunimmt und die Hydrophilie mit sinkender Temperatur zunimmt,
- 4,4',4"-(1,3,5-Triazin-2,4,6-triyltriimino)-tris-benzoesäure-tris(2-ethylhexylester),
- ferner gegebenenfalls weitere in der Ölphase lösliche oder dispergierbare Substanzen, darunter bevorzugt solche, gewählt aus der Gruppe der nicht unter die Definition des Emulgators A fallende Emulgatoren, insbesondere solche, die vorwiegend als W/O-Emulgatoren wirken,

zusammengegeben werden und unter Agitation ein Gemisch gebildet wird, dergestalt, daß

- dieses Gemisch durch geeignete Wahl der Temperatur in den Phaseninversionsbereich gebracht wird, in welchem sich W/O-Emulsionen in O/W-Emulsionen umwandeln,
- durch Variation der Temperatur die gebildete W/O-Emulsion aus dem Phaseninversionsbereich heraus gebracht wird, in welchem sich eine gebildete W/O-Emulsion in eine O/W-Emulsion umwandelt, wodurch eine O/W-Emulsion oder O/W-Mikroemulsion erzeugt wird
- das Gemisch gegebenenfalls weiteren Aufbereitungsschritten, insbesondere einem oder mehreren Homogenisierungsschritten unterworfen wird,

den Nachteilen des Standes der Technik abhelfen.

Erfindungsgemäß ist möglich, hohe Einsatzkonzentrationen in Ölkomponenten an sich schwerlöslicher UV-Filtersubstanzen, insbesondere des 4,4',4"-(1,3,5-Triazin-2,4,6-triyltriimino)-tris-benzoesäure-tris(2-ethylhexylesters) d. h., typischerweise etwa 5 Gew.%, bezogen auf das Gesamtgewicht einer O/W-Mikroemulsion mit einem Ölphasenanteil von etwa 50 Gew.-%, wieder bezogen auf das Gesamtgewicht der zubereitung, zu erreichen und damit die vorteilhaften Eigenschaften dieses Lichtschutzfilters voll auszunutzen.

Es ist durchaus vorteilhaft, auf weitere, nicht unter die Definition des Emulgators A fallende, weitere Emulgatoren, namentlich W/O-Emulgatoren, zu verzichten.

Erfindungsgemäß können O/W-Mikroemulsionen erhalten werden, wenn der Ölphasenanteil unter etwa 20 Gew.-%, insbesondere unter etwa 15 Gew.-%, bezogen auf das Gesamtgewicht der Zubereitung liegt, weniger als etwa 5 Gew.-% eines zusätzlichen, nicht unter die Definition des Emulgators A fallenden W/O-Emulgators vorliegen und/oder wenn die Ölphase einen hohen Anteil an polaren Ölen aufweist.

Erfindungsgemäß können O/W-Emulsionen ("Makroemulsionen") erhalten werden, wenn weniger als etwa 5 Gew.-% eines zusätzlichen nicht unter die Definition des Emulgators A fallenden W/O-Emulgators und mehr als etwa 20 Gew.-% einer polaren Ölphase vorliegen. Vorteilhaft können zusätzliche Gelbildner (z.B. Carbopole, Xanthangummi, Cellulosederivate) eingesetzt werden.

Es ist dabei im Einzelfalte möglich, daß die vorgenannten Konzentrationsgrenzen leicht über- oder unterschritten werden und dennoch die betreffenden Emulsionstypen erhalten werden. Dies kommt angesichts der breit streuenden Vielfalt an geeigneten Emulgatoren und Ölbestandteilen für den Fachmann nicht unerwartet, so daß er weiß, daß bei solchen Über- oder Unterschreitungen der Boden der vorliegenden Erfindung nicht verlassen wird.

Es hat sich in erstaunlicher Weise herausgestellt, daß der oder die erfindungsgemäß eingesetzten an sich schwerlöslichen UV-Filtersubstanzen vorzüglich in Lösung stabilisiert werden können und daß Rekristallisation unlöslicher oder schwerlöslicher UV-Filtersubstanzen verhindert werden kann. Darüberhinaus sind erfindungsgemäß Lichtschutzzubereitungen erhältlich, welche vorzügliche Anwendungseigenschaften aufweisen.

Erfindungsgemäß gleichermaßen vorteilhaft sind Verfahren, bei welchen die Variation des Parameters oder der Parameter darin besteht, daß

(a) bei vorgegebenem pH-Wert und vorgegebener Konzentration des Emulgators A bzw. der Vielzahl an Emulgatoren A die Temperatur des Gemisches sowie gegebenenfalls zusätzlich die Konzentration mindestens eines weiteren Emulgators A variiert wird, daß
(b) bei vorgegebener Konzentration mindestens eines Emulgators A der pH-Wert sowie zusätzlich die Temperatur des Gemisches sowie gegebenenfalls zusätzlich die Konzentration mindestens eines weiteren Emulgators A variiert werden.

Der Phaseninversionsbereich läßt sich mathematisch darstellen als Punktmenge innerhalb des geradlinigen Koordinatensystems Σ, welches durch die Größen Temperatur, pH-Wert und Konzentration eines geeigneten Emulgators bzw. eines Emulgatorengemisches in der Zubereitung gebildet wird, gemäß:

$$\Sigma = (O, \theta, a, m),$$

mit

O - Koordinatenursprung
θ - Temperatur
a - pH-Wert
m - Konzentration

Dabei muß genaugenommen natürlich in einem mehrkomponentigen Emulgatorensystem der Beitrag $m_i$ jedes einzelnen Emulgators zur Gesamtfunktion berücksichtigt werden, was bei einem i-komponentigen Emulgatorensystem zur Beziehung

$$\Sigma = \{O, \theta, a, m_1, m_2, ..., m_i\} \text{ führt.}$$

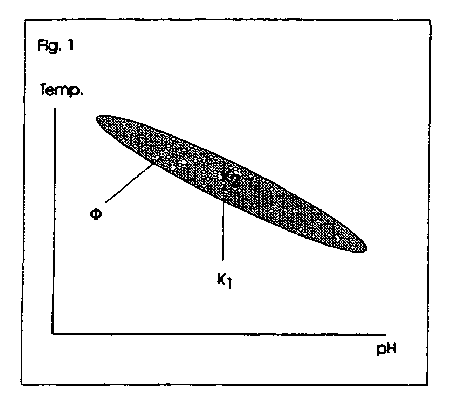

Der Phaseninversionsbereich Φ stellt dabei im mathematischen Sinne ein zusammenhängendes Gebiet oder eine Vielzahl zusammenhängenden Gebiete innerhalb des Koordinatensystems Σ dar. Φ repräsentiert die Gesamtmenge der Koordinatenpunkte $K(\theta, a, m_1, m_2, ..., m_i)$, welche erfindungsgemäße Gemische aus Wasser- und Ölphase, i erfindungsgemäßen Emulgatoren der Konzentration $m_i$ bei der Temperatur θ und dem pH-Wert a bestimmen, und für

welche gilt, daß beim Übergang von einer Koordinate $K_1 \notin \Phi$ zu einer Koordinate $K_2 \in \Phi$ Phaseninversion eintritt, wie in Fig. 1 beschrieben. Als variable Koordinaten in Fig.1 wurden Temperatur und pH-Wert eines gegebenen Gemisches unter Konstanthaltung der Konzentration von m, - $m_i$ angegeben. Beim Übergang von $K_1$ nach $K_2$ wird lediglich die Temperatur erhöht.

Unter den erfindungsgemäßen Bedingungen ist dieser Prozeß nicht notwendig reversibel, d.h., kehrt das System von der Koordinate $K_2 \in \Phi$ wieder zur Koordinate $K_1 \notin \Phi$ zurück, können erfindungsgemäß andere Emulsionstypen erhalten werden, als der Stand der Technik hätte erwarten lassen. Beispielsweise wird durch Erhöhen der Temperatur eines erfindungsgemäßen Gemisches aus Wasser- und Ölphase, i erfindungsgemäßen Emulgatoren der Konzentrationen $m_i$, wobei der pH-Wert a konstant bleibt, ausgehend von einer Temperatur welche für Phaseninversion zu niedrig ist (Bedingungen also, wo eine herkömmliche O/W-Emulsion vorläge, welche auch beim Abkühlen auf Raumtemperatur eine solche bliebe), erwärmt wird, so daß Phaseninversion eintritt, wird nach Abkühlen, z.B. auf Raumtemperatur, keine herkömmliche O/W-Emulsion erhalten, sondern eine erfindungsgemäße O/W-Mikroemulsion.

Unerheblich ist dabei, ob der Phaseninversionsbereich eines gegebenen Systems ein einziges zusammenhängendes (i + 2)-dimensionalen Gebiet darstellt oder aus mehreren zusammenhängenden, aber voneinander getrennten solchen Gebieten besteht, also mehreren Phaseninversionsbereichen eines gegebenen Systems entsprechend. Im Rahmen der hiermit vorgelegten Offenbarung wird daher stets verallgemeinernd von einem Phaseninversionsbereich gesprochen, auch bei Vorliegen zweier oder mehrerer voneinander getrennter solcher Bereiche.

Die Praxis der Herstellung einer erfindungsgemäßen Emulsion besteht vorteilhaft darin, nach Auswahl geeigneter Rohstoffe, d.h., Wasser- und Ölphase, ein oder mehrere Emulgatoren vorn Typ A, letzterer oder letztere vorliegend in Konzentrationen, bei weichen Phaseninversion für das gegebene Gemisch möglich ist, und gegebenenfalls weitere Substanzen, die Einzelkomponenten unter Agitation auf eine Temperatur zu erwärmen, bei welcher Phaseninversion für das gegebene Gemisch möglich ist, und durch Erhöhung oder Erniedrigung des pH-Wertes des Gemisches Phaseninversion herbeizuführen, hernach unter fortwährenden Agitation das Gemisch auf Raumtemperatur abkühlen zu lassen. Ein oder mehrere zwischengeschaltete Homogenisierungsschritte sind vorteilhaft, aber nicht zwingend notwendig.

In der Praxis ist möglich und gegebenenfalls sogar vorteilhaft, bei der Herstellung einer erfindungsgemäßen Emulsion den Temperaturbereich, der dem Phaseninversionsbereich zugeordnet werden kann auch zu übersteigen, da beim Abkühlen auf Raumtemperatur dieser Bereich dann zwangsläufig durchlaufen wird.

Grundsätzlich ist möglich und vorteilhaft, den 4,4',4"-(1,3,5-Triazin-2,4,6-triyltriimino)-tris-benzoesäure-tris (2-ethylhexylester) zu jedem beliebigen Zeitpunkt dem den Emulsionen zugrundeliegenden Gemisch zuzugeben. Es wird allerdings bevorzugt, diese Substanz bereits vor dem Emulgierungsvorgang in einem polarem Öl oder einem Gemisch aus polaren Ölen zu lösen und erst hernach das erfindungsgemäße Verfahren in Gang zu setzen. Es ist allerdings gegebenenfalls von Vorteil, den 4,4',4"-(1,3,5-Triazin-2,4,6-triyltriimino)-tris-benzoesäure-tris(2-ethylhexylester) unter Erwärmen in der polaren Ölphase zu lösen.

Die erfindungsgemäßen Emulsionen sind vorteilhaft dadurch gekennzeichnet, daß der Emulgator A oder die Emulgatoren A in Konzentrationen von 0,01 -20 Gew.-%, bevorzugt 0.05 - 10 Gew.-%, besonders bevorzugt 0,1 - 5 Gew.-%, jeweils bezogen auf das Gesamtgewicht der Zusammensetzung, vorliegt oder vorliegen.

Es ist erfindungsgemäß möglich die Einsatzmengen von 4,4',4"-(1,3,5-Triazin-2,4,6-triyltriimino)-tris-benzoesäure-tris(2-ethyihexylester) in kosmetischen oder dermatologischen Zubereitungen gegenüber dem Standes der Technik bis zu verdreifachen.

Ferner war erstaunlich, daß durch Zugabe erfindungsgemäßer Salicylsäurederivate eine Stabilisierung von Lösungen des 4,4',4"-(1,3,5-Triazin-2,4,6-triyltriimino)-tris-benzoesäure-tris(2-ethylhexylester) bewirkt wird, da die letztere Substanz nicht nur schlechte Löslichkeit aufweist, sondern auch aus seiner Lösung leicht wieder auskristallisiert. Erfindungsgemäß ist daher auch ein Verfahren zur Stabilisierung von Lösungen des 4,4',4"-(1,3,5-Triazin-2,4,6-triyltriimino)-tris-benzoesäure-tris(2-ethylhexylesters) in einer Ölphase, dadurch gekennzeichnet, daß solchen Lösungen in O/W-Mikroemulsionen vorliegen.

Insbesondere vorteilhaft im Sinne der vorliegenden Erfindung ist ein Verfahren zur Stabilisierung von 4,4', 4"-(1,3,5-Triazin-2,4,6-triyltriimino)-tris-benzoesäure-tris(2-ethylhexylester) in einer Ölphase, dadurch gekennzeichnet, daß der 4,4',4"-(1,3,5-Triazin-2,4,6-triyltrümino)-tris-benzoesäure-tris(2-ethylhexylester) dergestalt einer O/W-Mikroemulsion einverleibt wird, daß

- die Bestandteile einer Wasserphase,
- gegebenenfalls übliche, in Wasser lösliche oder dispergierbare Substanzen, die Bestandteile einer Ölphase
- mindestens ein Emulgator (Emulgator A), gewählt aus der Gruppe der Emulgatoren mit folgenden Eigenschaften
- ihre Lipophilie ist abhängig von der Temperatur, dergestalt, daß die Lipophilie mit steigender Temperatur zunimmt und die Hydrophilie mit sinkender Temperatur zunimmt.
- der 4,4',4"-(1,3,5-Triazin-2,4,6-triyltriimino)-tris-benzoesäure-tris(2-ethylhexylester),
- ferner gegebenenfalls weitere in der Ölphase lösliche oder dispergierbare Substanzen, darunter bevorzugt solche,

gewählt aus der Gruppe der nicht unter die Definition des Emulgators A fallende Emulgatoren, insbesondere solche, die vorwiegend als W/O-Emulgatoren wirken,

zusammengegeben werden und unter Agitation ein Gemisch gebildet wird, dergestalt, daß

- dieses Gemisch durch geeignete Wahl der Temperatur in den Phaseninversionsbereich gebracht wird, in welchem sich W/O-Emulsionen in O/W-Emulsionen umwandeln,
- durch Variation der Temperatur die gebildete W/O-Emulsion aus dem Phaseninversionsbereich heraus gebracht wird, in welchem sich eine gebildete W/O-Emulsion in eine O/W-Emulsion umwandelt, wodurch eine O/W-Emulsion oder O/W-Mikroemulsion erzeugt wird,
- das Gemisch gegebenenfalls weiteren Aufbereitungsschritten, insbesondere einem oder mehreren Homogenisierungsschritten unterworfen wird.

Besonders bevorzugt sind erfindungsgemäße Zubereitungen, welche als weitere UV-Schutzsubstanzen Salicylsäurederivate 4-Isopropylbenzylsalicylat, 2-Ethylhexylsalicylat, (Octylsalicylat) und/oder Homomenthylsalicylat enthalten, insbesondere solcher Salicylsäurederivate der allgemeinen Struktur

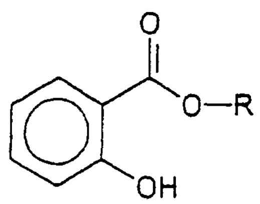

wobei R darstellen kann:

(1) einen verzweigten oder unverzweigten Alkylrest mit 5 - 18 Kohlenstoffatomen oder
(2) einen unsubstituierten Cycloalkylrest mit 5 - 7 Kohlenstoffatomen oder
(3) einen mit bis zu 10 verzweigten oder unverzweigten Alkylresten mit 5 - 18 Kohlenstoffatomen substituierten Cycloalkylrest mit 5 - 7 Kohlenstoffatomen oder
(4) einen unsubstituierten Phenylrest oder
(5) einen mit bis zu 5 verzweigten oder unverzweigten Alkylresten mit 5 - 18 Kohlenstoffatomen substituierten Phenylrest.

Zu gebräuchlichen UV-Filtern gehören darunter

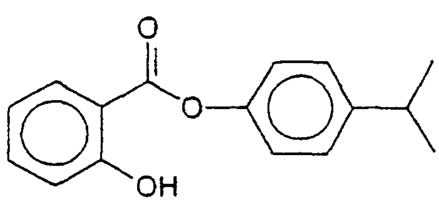

(4-Isopropylbenzylsalicylat),

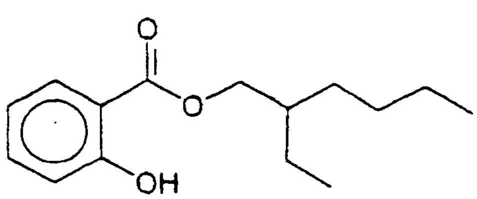

(2-Ethylhexylsalicylat, Octylsalicylat),

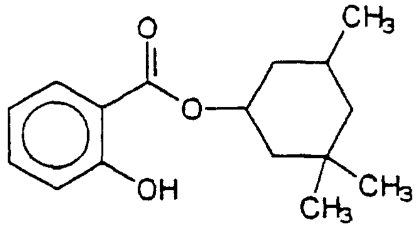

(Homomenthylsalicylat).

Die Gesamtmenge an in Ölkomponenten an sich schwerlöslichen UV-Filtersubstanzen, insbesondere 4,4', 4"-(1,3,5-Triazin-2.4,6-triyftriimino)-tris-benzoesäure-tris(2-ethylhexylester) in den fertigem kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an einem oder mehreren erfindungsgemäßen Salicylsäurederivaten in den fertigem kosmetischen oder dermatologischen Zubereitungen wird vorteilhaft aus dem Bereich von 0,1 - 10,0 Gew.-%, bevorzugt 0,5 - 6,0 Gew.-% gewählt, bezoge auf das Gesamtgewicht der Zubereitungen.

Es ist von Vorteil, Gewichtsverhältnisse von 4,4',4"-(1,3,5-Triazin-2,4,6-triyltriimino)-trisbenzoesäure-tris (2-ethylhexylester) und einem oder mehreren erfindungsgemäßen Salicylsäurederivaten aus dem Bereich von 1 : 10 bis 10 : 1, bevorzugt 1 : 4 bis 4 : 1, zu wählen.

Es ist erfindungsgemäß vorteilhaft, in den erfindungsgemäßen Zubereitungen weitere öllösliche UVA-Filter und/oder UVB-Filter in der Lipidphase und/oder wasserlösliche UVA-Filter und/oder UVB-Filter in der wäßrigen Phase einzusetzen.

Vorteilhaft können die erfindungsgemäßen Lichtschutzformulierungen weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen.

Die UVB-Filter können öllöslich oder wasserlöslich sein. Vorteilhafte öllösliche UVB-Filtersubstanzen sind z. B.:

- 3-Benzylidencampher-Derivate, vorzugsweise 3-(4-Methylbenzyliden)campher, 3-Benzylidencampher,
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;

Vorteilhafte wasserlösliche UVB-Filtersubstanzen sind z.B.:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-methoxybenzophenon-5-sulfonsäure und deren Salze;
- Sulfonsäure-Derivate des 3-Benzylidencamphers, wie z.B. 4-(2-Oxo-3-bomylidenmethyl)benzalsulfonsäure,2-Methyl-5-(2-oxo-3-bornylidenmethyl)sulfonsäure und deren Salze.

Die Liste der genannten UVB-Filter, die in Kombination mit den erfindungsgemäßen Wirkstoffkombinationen verwendet werden können, soll selbstverständlich nicht limitierend sein.

Es kann auch von Vorteil sein, UVA-Filter in den erfindungsgemäßen Zubereitungen einzusetzen, welche bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Ferner ist vorteilhaft, die erfindungsgemäßen Wirkstoffkombinationen mit weiteren UVA-und/oder UVB-Filtern

zu kombinieren.

Erfindungsgemäße kosmetische und dermatologische Zubereitungen enthalten vorteilhaft außerdem anorganische Pigmente auf Basis von Metalloxiden und/oder anderen in Wasser schwerlöslichen oder unlöslichen Metallverbin. dungen, insbesondere der Oxide des Titans ($TiO_2$), Zinks (ZrO), Eisens (z.B. $Fe_2O_3$), Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Es ist besonders vorteilhaft im Sinne der vorliegenden Erfindung, wenngleich nicht zwingend, wenn die anorganischen Pigmente in hydrophober Form vorliegen, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Rektion gemäß

$$n\ TiO_2 + m\ (RO)_3\ Si\text{-}R' \rightarrow n\ TiO_2\ (\text{oberfl.})$$

erzeugt wird, n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen MT 100 T von der Firma TAYCA erhältlich.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme. Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D, L-Carnosin. D-Carnosin, L-Carnosin und deren Derivate (z.B. Anserin), Carotinoide, Carotine (z.B. $\alpha$-Carotin, $\beta$-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis $\mu$mol/kg), ferner (Metall)-Chelatoren (z.B. $\alpha$-Hydroxyfettsäuren, Palmitinsäure, Phytinsäure, Lactoferrin), $\alpha$-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. $\gamma$-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, $\alpha$-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZrO, $ZnSO_4$) Selen und dessen Derivate (z.B. Selenmethionin), Stilbene und deren Derivate (z.B. Stilbenoxid, Trans-Stilbenoxid) und die erfindungsgemäß

geeigneten Derivate (Salze, Ester, Ether, Zukker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Lipidphase kann vorteilhaft gewählt werden aus folgender Substanzgruppe:

- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol. Ethylenglykolmonoethyl- oder -monobutylether, Propylenglykolmonomethyl, -monoethyl- oder - monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die kosmetischen oder dermatologischen Lichtschutzzubereitungen enthalten vorteilhaft anorganische Pigmente, insbesondere Mikropigmente, z.B. in Mengen von 0,1 Gew.-% bis 30 Gew.-%, vorzugsweise in Mengen von 0,5 Gew.-% bis 10 Gew.-%, insbesondere aber 1 Gew.-% bis 6 Gew.-%, bezogen auf das Gesamtgewicht der Zubereitungen.

Nachfolgend soll kurz noch auf einige Besonderheiten und Unterschiede der Voraussetzungen für erfindungsgemäß hergestellte O/W-Emulsionen, und O/W-Mikroemulsionen eingegangen werden.

Öle und Fette unterscheiden sich unter anderem in ihrer Polarität, welche schwierig zu definieren ist. Es wurde bereits vorgeschlagen, die Grenzflächenspannung gegenüber Wasser als Maß für den Polaritätsindex eines Öls bzw. einer Ölphase anzunehmen. Dabei gilt, daß die Polarität der betreffenden Ölphase umso größer ist, je niedriger die Grenzflächenspannung zwischen dieser Ölphase und Wasser ist. Erfindungsgemäß wird die Grenzflächenspannung als ein mögliches Maß für die Polarität einer gegebenen Ölkomponente angesehen.

Die Grenzflächenspannung ist diejenige Kraft, die an einer gedachten, in der Grenzflache zwischen zwei Phasen befindlichen Linie der Länge von einem Meter wirkt. Die physikalische Einheit für diese Grenzflächenspannung errechnet sich klassisch nach der Beziehung Kraft/Länge und wird gewöhnlich in mN/m (Millinewton geteilt durch Meter) wiedergegeben. Sie hat positives Vorzeichen, wenn sie das Bestreben hat, die Grenzfläche zu verkleinern. Im umgekehrten Falle hat sie negatives Vorzeichen.

Als Grenze, unterhalb derer eine Ölphase als "polar" und oberhalb derer eine Ölphase als "unpolar" gilt, werden erfindungsgemäß 30 mN/m angesehen.

Erfindungsgemäß wird für O/W-Mikroemulsionen die Ölphase vorteilhaft gewählt aus der Gruppe der polaren Ölkomponenten, welche eine Polarität zwischen 10 und 30 mN/m aufweisen, wobei gewährleistet sein muß, daß mindestens eine nichtpolare Ölkomponente vorhanden ist.

Vorteilhafte O/W-Mikroemulsionen werden erhalten, wenn die Ölphase gewählt wird aus der Gruppe der polaren Ölkomponenten, besonders bevorzugt aus der Gruppe der natürlichen, synthetischen oder halbsynthetischen Ölkomponenten, welche eine Polarität zwischen 10 und 20 mN/m aufweisen, wobei gewährleistet sein muß, daß mindestens eine nichtpolare Ölkomponente vorhanden ist.

Vorteilhaft ist auch, polare pflanzliche Öle als polare Öle der erfindungsgemäßen O/W-Emulsionen zu verwenden. Die pflanzlichen Öle können vorteilhaft gewählt werden aus der Gruppe der Öle der Pflanzenfamilien Euphorbiaceae, Poaceae, Fabaceae, Brassicaceae, Pedalaceae, Asteraceae, Linaceae, Flacourticaceae, Violales, vorzugsweise gewählt aus der Gruppe natives Rizinusöl, Weizenkeimöl, Traubenkernöl, Kukuinußöl, Safloröl, Distelöl, Nachtkerzenöl und weiteren Ölen, welche mindestens 1,5 Gew.-% an Linolsäureglyceriden enthalten.

Die erfindungsgemäßen Mikroemulsionen enthalten daher vorteilhaft Elektrolyte, insbesondere eines oder mehrere Salze mit folgenden Anionen: Chloride, ferner anorganische Oxo-Element-Anionen, von diesen insbesondere Sulfate, Carbonate, Phosphate, Borate und Aluminate. Auch auf organischen Anionen basierende Elektrolyte können vorteilhaft verwendet werden, beispielsweise Lactate, Acetate, Benzoate, Propionate, Tartrate, Citrate und andere mehr. Vergleichbare Effekte sind auch durch Ethylendiamintetraessigsäure und deren Salze zu erzielen.

Als Kationen der Salze werden bevorzugt Ammonium,- Alkylammonium,- Alkalimetall-, Erdalkalimetall,- Magnesium-, Eisen- bzw. Zinkionen verwendet. Es bedarf an sich keiner Erwähnung, daß in Kosmetika nur physiologisch unbedenkliche Elektrolyte verwendet werden sollten. Spezielle medizinische Anwendungen der erfindungsgemäßen Mikroemulsionen können andererseits, wenigstens grundsätzlich, die Verwendung von Elektrolyten bedingen, welche nicht ohne ärztliche Aufsicht verwendet werden sollten.

Besonders bevorzugt sind Kaliumchlorid, Kochsalz. Magnesiumsulfat, Zinksulfat und Mischungen daraus. Ebenfalls vorteilhaft sind Salzmischungen wie sie im natürlichen Salz vom Toten Meer auftreten.

Die Konzentration des Elektrolyten oder der Elektrolyte sollte etwa 0,01 - 10,0 Gew.-%, besonders vorteilhaft etwa 0,03 - 8,0 Gew.% betragen, bezogen auf das Gesamtgewicht der Zubereitung.

Die Emulgatoren des Typs A können landsläufig als O/W-Emulgatoren angesehen werden.

Dabei liegt im allgemeinen Fachwissen des Fachmannes und bedarf keinerlei erfinderischen Dazutuns, für einen gegebenen Emulgator oder ein gegebenes Emulgatorsystem in einem gegebenen Wasser/Ölphasensystem den Temperatur bereich zu ermitteln, in welchem Phaseninversion stattfindet. Als allgemeinen Richtwert für den PIT bei üblichen Emulgatorkonzentrationen kann ein Temperaturbereich von etwa 40 - 90° C angegeben werden. Im allgemeinen sinkt der PIT bei steigender Emulgatorkonzentration.

Es können während dieses Verfahrens gewünschtenfalls ferner die in der Kosmetik oder medizinischen Galenik üblichen Grund-, Hilfs-, Zusatz-, und/oder Wirkstoffe zugegeben werden. Es ist dem Fachmann klar, zu welchem Zeitpunkt solche Stoffe dem Prozeß beigegeben werden können, ohne daß die Eigenschaften der zu erzielenden Emulsion wesentlich beeinträchtigt werden.

Die nachfolgenden Beispiele sollen das Wesen der vorliegenden Erfindung näher umreißen, ohne die Erfindung einzuschränken.

**Beispiel 1**

| O/W-Mikroemulsion | |
|---|---|
| | Gew.-% |
| Ceteareth-12 | 8,00 |
| Cetearylisononanoat | 20,00 |
| Cetearylalkohol | 4,00 |
| Uvinul® T150 | 2,00 |
| Parsol® 1789 | 1,00 |
| Eusolex® 232 | 4,80 |
| | pH=7,5 |
| Farbstoffe, Parfum, Konservierungsmittel | q.s. |
| Wasser | ad 100,00 |

Der UV-Filter wird in der der Ölphase gelöst und mit den übrigen Bestandteilen der Ölphase vereinigt, worauf das Gemisch homogenisiert, dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht).

**Beispiel 2**

| O/W-Mikroemulsion | |
|---|---|
| | Gew.-% |
| Ceteareth-12 | 12,00 |
| Cetearylisononanoat | 20,00 |
| Cetearylalkohol | 6,00 |
| Uvinul® T150 | 2,00 |
| Eusolex® 6300 | 3,00 |
| Eusolex® 232 | 4,80 |
| | pH=7,5 |
| Farbstoffe, Parfum, Konservierungsmittel | q.s. |
| Wasser | ad 100,00 |

Der UV-Filter wird in der der Ölphase gelöst und mit den übrigen Bestandteilen der Ölphase vereinigt, worauf das Gemisch homogenisiert, dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht).

**Beispiel 3**

| O/W-Mikroemulsion | |
|---|---|
| | Gew.-% |
| Ceteareth-12 | 8,00 |
| Cetearylisononanoat | 20,00 |
| Cetearylalkohol | 4,00 |
| Uvinul® T150 | 4,80 |
| $MgSO_4$ | 3,00 |
| Farbstoffe, Parfum, Konservierungsmittel | q.s. |
| Wasser | ad 100,00 |

Der UV-Filter wird in der der Ölphase gelöst und mit den übrigen Bestandteilen der Ölphase vereinigt, worauf das Gemisch homogenisiert, dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht).

**Beispiel 4**

| O/W-Mikroemulsion | |
|---|---|
| | Gew.-% |
| Ceteareth-12 | 12,00 |
| Cetearylisononanoat | 20,00 |
| Cetearylalkohol | 6,00 |
| Uvinul® T150 | 4,80 |
| Parsol® 1789 | 2,00 |
| $TiO_2$ | 2,00 |

(fortgesetzt)

| O/W-Mikroemulsion | |
|---|---|
| | Gew.-% |
| $MgSO_4$ | 3,00 |
| Farbstoffe, Parfum, Konservierungsmittel | q.s. |
| Wasser | ad 100,00 |

Der UV-Filter wird in der der Ölphase gelöst und mit den übrigen Bestandteilen der Ölphase vereinigt, worauf das Gemisch homogenisiert, dann mit der Wasserphase vereinigt und auf eine Temperatur von 80 - 85° C (d.h., in den Phaseninversionstemperaturbereich des Systems) gebracht hernach auf Raumtemperatur abgekühlt (also aus dem Phaseninversionstemperaturbereich des Systems wieder heraus gebracht).

**Beispiel 5**

| O/W-Mikroemulsion | |
|---|---|
| | Gew.-% |
| Ceteareth-12 | 8,00 |
| Cetearylisononanoat | 10,00 |
| Cetearylalkohol | 4,00 |
| Eusolex® 232 | 5,00 |
| Uvinul® T150 | 1,00 |
| Parsol® 1789 | 4,00 |
| | pH=7,5 |
| Farbstoffe, Parfum, Konservierungsmittel | q.s. |
| Wasser | ad 100,00 |

Der UV-Filter wird in der der Ölphase gelöst und mit den übrigen Bestandteilen der Ölphase vereinigt, worauf das Gemisch homogenisiert, dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht).

**Patentansprüche**

**1.** Verfahren zur Einarbeitung von 4,4',4"-(1,3,5-Triazin-2,4,6-triyltriimino)-tris-benzoesäure-tris(2-ethylhexylester) in O/W-Emulsionen, insbesondere O/W-Mikroemulsionen, **dadurch gekennzeichnet, daß**

- die Bestandteile einer Wasserphase,
- gegebenenfalls übliche, in Wasser lösliche oder dispergierbare Substanzen,
- die Bestandteile einer Ölphase
- mindestens ein Emulgator (Emulgator A) dessen Lipophilie von der Temperatur abhängig ist, dergestalt, daß die Lipophilie mit steigender Temperatur zunimmt und die Hydrophilie mit sinkender Temperatur zunimmt,
- 4,4',4"-(1,3,5-Triazin-2,4,6-triyltriimino)-tris-benzoesäure-tris(2-ethylhexylester)
- ferner gegebenenfalls weitere in der Ölphase lösliche oder dispergierbare Substanzen, darunter bevorzugt solche, gewählt aus der Gruppe der nicht unter die Definition des Emulgators A fallende Emulgatoren, insbesondere solche, die vorwiegend als W/O-Emulgatoren wirken,

zusammengegeben werden und unter Agitation ein Gemisch gebildet wird, dergestalt, daß

- dieses Gemisch durch geeignete Wahl der Temperatur in den Phaseninversionsbereich gebracht wird, in welchem sich W/O-Emulsionen in O/W-Emulsionen umwandeln,

- durch Variation der Temperatur die gebildete W/O-Emulsion aus dem Phaseninversionsbereich heraus gebracht wird, in welchem sich eine gebildete W/O-Emulsion in eine O/W-Emulsion umwandelt, wodurch eine O/W-Emulsion oder O/W-Mikroemulsion erzeugt wird
- das Gemisch gegebenenfalls weiteren Aufbereitungsschritten, insbesondere einem oder mehreren Homogenisierungsschritten unterworfen wird.

**2.** Verfahren nach Anspruch 1, **dadurch gekennzeichnet, daß** der 4,4',4"-(1,3,5-Triazin-2,4,6-triyltriimino)-tris-benzoesäure-tris(2-ethylhexylester) bereits vor dem Emulgierungsvorgang in einem polarem Öl oder einem Gemisch aus polaren Ölen gelöst werden und erst hernach die Verfahrensschritte gemäß Anspruch 1 durchgeführt werden.

**3.** O/W-Mikroemulsionen, erhältlich nach Anspruch 1 oder 2

**4.** O/W-Mikroemulsionen nach Anspruch 3, **dadurch gekennzeichnet, daß** der Emulgator A oder die Emulgatoren A in Konzentrationen von 0,01 - 20 Gew.-%, bevorzugt 0,05 - 10 Gew.-%, besonders bevorzugt 0,1 - 5 Gew.-%, jeweils bezogen auf das Gesamtgewicht der Zusammensetzung, vorliegt oder vorliegen.

## Claims

**1.** Process for incorporation of 4,4',4"-(1,3,5-triazine-2,4,6-triyltriimino)tris-benzoic acid tris-(2-ethylhexyl ester) into O/W emulsions, in particular O/W microemulsions, **characterized in that**

- the constituents of an aqueous phase,
- if appropriate, customary water-soluble or - dispersible substances,
- the constituents of an oily phase,
- at least one emulsifier (emulsifier A) whose lipophilicity depends on the temperature such that the lipophilicity increases with increasing temperature and the hydrophilicity increases with decreasing temperature,
- 4,4',4"-(1,3,5-triazine-2,4,6-triyltriimino)tris-benzoic acid tris(2-ethylhexyl ester),
- and furthermore, if appropriate, further substances which are soluble or dispersible in the oily phase, including, preferably, those chosen from the group of emulsifiers which do not fall under the definition of emulsifier A, in particular those which chiefly act as W/O emulsifiers,

are brought together and, with agitation, a mixture is formed such that

- by suitable choice of the temperature, this mixture is brought into the phase inversion range in which W/O emulsions are converted into O/W emulsions,
- by varying the temperature, the W/O emulsion formed is brought out of the phase inversion range in which a W/O emulsion formed is converted into an O/W emulsion, whereupon an O/W emulsion or O/W microemulsion is produced,
- if appropriate, the mixture is subjected to further processing steps, in particular one or more homogenizing steps.

**2.** Process according to Claim 1, **characterized in that** the 4,4', 4"-(1,3,5-triazine-2,4,6-triyltriimino)tris-benzoic acid tris(2-ethylhexylester) are already dissolved in a polar oil or a mixture of polar oils before the emulsifying operation and only then are the process steps according to the Claim 1 carried out.

**3.** O/W microemulsions obtainable according to Claim 1 or 2.

**4.** O/W microemulsions according to Claim 3, **characterized in that** the emulsifier A or the emulsifiers A is or are present in concentrations of 0.01-20 % by weight, preferably 0.05-10 % by weight, particularly preferably 0.1-5 % by weight, in each case based on the total weight of the composition.

## Revendications

**1.** Procédé pour l'incorporer l'ester tris(2-éthylhexylique) de l'acide 4,4',4"-(1,3,5-triazine-2,4,6-triyltriimino)-tris-benzoïque dans des émulsions H/E, en particulier des micro-émulsions H/E, **caractérisé en ce que**

- les constituants d'une phase aqueuse
- le cas échéant des substances usuelles solubles ou dispersibles dans l'eau
- les constituants d'une phase huileuse
- au moins un émulsifiant (émulsifiant A), dont la lipophilie dépend de la température, de telle manière que la lipophilie augmente avec l'augmentation de la température et l'hydrophilie augmente avec la diminution de la température,
- l'ester tris(2-éthylhexylique) de l'acide 4,4',4"-(1,3,5-triazine-2,4,6-triyltriimino)-tris-benzoïque,
- en outre le cas échéant d'autres substances solubles ou dispersibles dans la phase huileuse, parmi lesquelles en particulier celles choisies dans le groupe des émulsifiants ne tombant pas sous la définition de l'émulsifiant A, en particulier ceux qui agissent principalement comme émulsifiants E/H,

sont rassemblés et un mélange est formé sous agitation, de telle manière que

- ce mélange est amené dans la plage d'inversion de phase par un choix approprié de la température, dans laquelle les émulsions E/H se transforment en émulsions H/E,
- par la variation de la température, l'émulsion E/H formée est sortie de la plage d'inversion de phase dans laquelle une émulsion E/H formée se transforme en une émulsion H/E, suite à quoi on obtient une émulsion H/E ou une micro-émulsion H/E,
- le mélange est le cas échéant soumis à d'autres étapes de transformation, en particulier une ou plusieurs étapes d'homogénéisation.

2. Procédé selon la revendication 1, **caractérisé en ce que** l'ester tris(2-éthylhexylique) de l'acide 4,4',4"-(1,3,5-triazine-2,4,6-triyltriimino)-tris-benzoïque est déjà dissous avant le processus d'émulsion dans une huile polaire ou un mélange d'huiles polaires et ce n'est qu'ensuite que les étapes de procédé selon la revendication 1 sont réalisées.

3. Micro-émulsions H/E, pouvant être obtenues selon la revendication 1 ou 2.

4. Micro-émulsions H/E selon la revendication 3, **caractérisées en ce que** l'émulsifiant A ou les émulsifiants A sont utilisés en des concentrations de 0,01 à 20% en poids, de préférence de 0,05 à 10% en poids, de manière particulièrement préférée de 0,1 à 5% en poids, à chaque fois par rapport au poids total de la composition.

Fig. 1